# EUROPEAN PATENT APPLICATION

(11) **EP 1 275 398 A1**
(43) Date of publication of application: **15.01.2003**
(21) Application number: 01919804.3
(22) Date of filing: 06.04.2001
(51) Int. Cl.: A61K 45/00, A61K 39/395, A61K 48/00, A61P 29/00, A61P 19/02, A61P 43/00, G01N 33/53

(54) **DIAGNOSTICS AND REMEDIES FOR RHEUMATOID ARTHRITIS**

(30) Priority: 06.04.2000 JP 2000105087
(71) Applicant: KYOWA HAKKO KOGYO CO., LTD., Chiyoda-ku, Tokyo 100-8185 (JP)
(72) Inventor: HOMMA, Yoshimi, Fukushima-shi, Fukushima 960-8157 (JP); SATO, Koichiro, Fukushima-shi, Fukushima 960-8055 (JP)
(74) Representative: VOSSIUS & PARTNER
(86) International application number: JP0102981
(87) International publication number: WO01076630

(57) **Abstract**

The present invention provides a therapeutic agent for rheumatoid arthritis which inhibits the growth of synoviocytes mediated by c-erbB-2 by clarifying the participation of c-erbB-2 in the growth of synoviocytes in rheumatoid arthritis patients and inhibiting the activation or expression of c-erbB-2.

## Description

### Technical Field

The present invention relates to diagnostic and therapeutic agents for rheumatoid arthritis which inhibit the growth of synoviocytes mediated by c-erbB-2, and a diagnostic method for rheumatoid arthritis which comprises detecting c-erbB-2.

### Background Art

Rheumatoid arthritis is a systematic, chronic inflammatory disease characterized by the growth of synoviocytes at joints and the abnormality of immunoreaction, which gradually leads to the collapse of tendon, cartilage and bone tissues [Ann. Rheum. Dis., 52, S39-S47 (1993)].

So far, little has been elucidated about the signal transduction for excessive growth of synoviocytes in rheumatoid arthritis; however, the participation of growth factors such as EGF (epidermal growth factor), PDGF (platelet-derived growth factor) and FGF (fibroblast growth factor) which are present in synovia has been suggested [The Journal of Orthopaedic Science, 67, 859-865 (1993), Semin. Arthritis Rheum., 21, 191-199 (1991)]. There are also reports on the elevated expression levels of PDGF receptor in synoviocytes [Scand. J. Immnol., 27, 285-294 (1988)] and FGF receptor in focal T cells [Arthritis Rheum., 39, 914-922 (1996)]. However, little has been reported about EGF receptor (hereinafter referred to as EGFR) in tissues affected by rheumatoid arthritis.

EGFRs bind to EGF to form EGFR'S dimer and are activated, thereby leading to the cell growth. It is reported that, besides EGFR, c-erbB-2 (referred to also as HER2 or c-neu) of the erbB family having homology to EGFR forms a heterodimer with EGFR in the presence of EGF, and as a result, EGFR is activated as tyrosine kinase and c-erbB-2 is phosphorylated [Biochemistry, 29, 11024-11028 (1990)]. It is also reported that c-erbB-2 forms heterodimers with erbB-3 or erbB-4 which belong to the same erbB family and is activated by binding to neuregulin family members such as heregulin [Science, 256, 1205-1210 (1992); Mol. Cell. Biol., 15, 5770-5776 (1995); J. Biol. Chem., 269, 14661-14665 (1994); Nature, 366, 473-475 (1993)]. There has so far been found no ligand that directly binds to c-erbB-2 to activate c-erbB-2.

It is reported that c-erbB-2 has the ability to transform rat fibroblast cell line NIH3T3 when excessively expressed therein [Science, 237, 178-182 (1987); Proc. Natl. Acad. Sci. USA, 84, 7159-7163 (1987)] and that c-erbB-2 is usually expressed only at low levels in epithelial cells of various normal tissues of human adults [Oncogene, 5, 953-962 (1990)], whereas it is excessively expressed in 20 to 30% of lung cancers [Science, 235, 177-82 (1987); Cancer Lett., 81, 137-44 (1994); Cancer, 73, 2359-65 (1994); Science, 229, 974-6 (1985)], gastric cancers [Cancer Res., 50, 8002-9 (1990)], ovarian cancers [Science, 244, 707-12 (1989)] and pancreatic cancers [Pathology, 59, 46-72 (1991)]. These reports suggest the participation of c-erbB-2 in the abnormal cell growth such as cancer. Further, it is reported that the growth of cancer cells is suppressed by antibodies to c-erbB-2 [Mol. Cell. Biol., 9, 1165-1172 (1989); U.S. Patent No. 5677171; PCT National Publication No. 504172/96; Int. J. Can., 53, 401-408 (1993); Am. J. Resp. Cell. Mol. Biol., 9, 448-454 (1993); Clin. Ther., 21, 309-318 (1999)], a single-chain antibody to the intracellular domain of c-erbB-2 which causes down regulation of c-erbB-2 [Gene Ther., 1, 332-337 (1994)] and antisense nucleotides [PCT National Publication No. 506770/97; Br. J. Can., 70, 819-125 (1994); Int. J. Can., 72, 631-636 (1997)], but no report has been made on their participation in the growth of synoviocytes in rheumatoid arthritis.

### Disclosure of the Invention

An object of the present invention is to provide a therapeutic agent for rheumatoid arthritis which inhibits the growth of synoviocytes mediated by c-erbB-2 by clarifying the participation of c-erbB-2 in the growth of synoviocytes in rheumatoid arthritis patients and inhibiting the activation or expression of c-erbB-2.

On the basis of the hypothesis that the main cause of the excessive growth of synoviocytes in rheumatoid arthritis patients is activation of c-erbB-2, the present inventors have demonstrated that a large quantity of c-erbB-2 is present on synoviocytes in rheumatoid arthritis patients and have found that c-erbB-2 is phosphorylated and activated by addition of EGF. The inventors have further found that the growth of synoviocytes is inhibited by addition of genistein, which is a tyrosine kinase inhibitor, or anti-c-erbB-2 antibody, and have completed the present invention.

The present invention relates to the following (1) to (27).
(1) A therapeutic agent for rheumatoid arthritis comprising, as an active ingredient, a substance which inhibits the biological activities of c-erbB-2.
(2) The therapeutic agent for rheumatoid arthritis according to the above (1), wherein the substance which inhibits the biological activities of c-erbB-2 is a substance which inhibits the signal transduction mediated by c-erbB-2 or a substance which suppresses the expression of c-erbB-2.
(3) The therapeutic agent for rheumatoid arthritis according to the above (2), wherein the substance which inhibits the signal transduction mediated by c-erbB-2 is at least one member selected from the group consisting of substances which inhibit the binding of an erbB ligand to c-erbB-2, substances which inhibit the binding of a c-erbB-2 ligand to a receptor comprising c-erbB-2, substances which inhibit the dimerization of c-erbB-2, and substances which inhibit the signal transduction after the binding of an erbB ligand to c-erbB-2.
(4) The therapeutic agent for rheumatoid arthritis according to the above (2), wherein the substance which inhibits the signal transduction mediated by c-erbB-2 is at least one member selected from the group consisting of neutralizing antibodies recognizing c-erbB-2, fragments of said antibodies, and derivatives thereof.
(5) The therapeutic agent for rheumatoid arthritis according to the above (3), wherein the substances which inhibit the binding of an erbB ligand to c-erbB-2 or the substances which inhibit the binding of an erbB ligand to a receptor comprising c-erbB-2 are c-erbB-2 antagonists or antagonists of receptors forming heteroreceptors with c-erbB-2.
(6) The therapeutic agent for rheumatoid arthritis according to the above (3), wherein the substances which inhibit the binding of an erbB ligand to c-erbB-2 or the substances which inhibit the binding of an erbB ligand to a receptor comprising c-erbB-2 are substances which bind to an erbB ligand and inhibit the binding of the erbB ligand to c-erbB-2 or a receptor forming a heteroreceptor with c-erbB-2.
(7) The therapeutic agent for rheumatoid arthritis according to the above (5), wherein the antagonists of c-erbB-2 or receptors forming heteroreceptors with c-erbB-2 are neutralizing antibodies recognizing c-erbB-2 or receptors forming heteroreceptors with c-erbB-2, fragments of said antibodies, or derivatives thereof.
(8) The therapeutic agent for rheumatoid arthritis according to the above (6), wherein the substances which bind to an erbB ligand and inhibit the binding of the erbB ligand to c-erbB-2 or a receptor forming a heteroreceptor with c-erbB-2 are neutralizing antibodies recognizing erbB ligands, fragments of said antibodies, or derivatives thereof.
(9) The therapeutic agent for rheumatoid arthritis according to the above (3), wherein the substances which inhibit the signal transduction after the binding of an erbB ligand to c-erbB-2 are substances which inhibit the tyrosine phosphorylation of c-erbB-2 or substances which inhibit the interaction between phosphorylated c-erbB-2 and an intracellular signaling molecule.
(10) The therapeutic agent for rheumatoid arthritis according to the above (9), wherein the substances which inhibit the tyrosine phosphorylation of c-erbB-2 are tyrosine kinase inhibitors.
(11) The therapeutic agent for rheumatoid arthritis according to the above (9), wherein the substances which inhibit the interaction between phosphorylated c-erbB-2 and an intracellular signaling molecule are antibodies to the intracellular domain of c-erbB-2, fragments of said antibodies, or derivatives thereof.
(12) The therapeutic agent for rheumatoid arthritis according to the above (2), wherein the substance which suppresses the expression of c-erbB-2 is at least one member selected from the group consisting of antisense DNAs, antisense oligonucleotides, triple helix-forming oligonucleotides and ribozymes for the c-erbB-2 gene.
(13) A synoviocyte growth inhibitor comprising, as an active ingredient, a substance which inhibits the biological activities of c-erbB-2.
(14) The synoviocyte growth inhibitor according to the above (13), wherein the substance which inhibits the biological activities of c-erbB-2 is a substance which inhibits the signal transduction mediated by c-erbB-2 or a substance which suppresses the expression of c-erbB-2.
(15) The synoviocyte growth inhibitor according to the above (14), wherein the substance which inhibits the signal transduction mediated by c-erbB-2 is at least one member selected from the group consisting of substances which inhibit the binding of an erbB ligand to c-erbB-2, substances which inhibit the binding of a c-erbB-2 ligand to a receptor comprising c-erbB-2, substances which inhibit the dimerization of c-erbB-2, and substances which inhibit the signal transduction after the binding of an erbB ligand to c-erbB-2.
(16) The synoviocyte growth inhibitor according to the above (14), wherein the substance which inhibits the signal transduction mediated by c-erbB-2 is at least one member selected from the group consisting of neutralizing antibodies recognizing c-erbB-2, fragments of said antibodies, and derivatives thereof.
(17) The synoviocyte growth inhibitor according to the above (15), wherein the substances which inhibit the binding of an erbB ligand to c-erbB-2 or the substances which inhibit the binding of an erbB ligand to a receptor comprising c-erbB-2 are c-erbB-2 antagonists or antagonists of receptors forming heteroreceptors with c-erbB-2.
(18) The synoviocyte growth inhibitor according to the above (15), wherein the substances which inhibit the binding of an erbB ligand to c-erbB-2 or the substances which inhibit the binding of a c-erbB-2 ligand to a receptor comprising c-erbB-2 are substances which bind to an erbB ligand and inhibit the binding of the erbB ligand to c-erbB-2 or a receptor forming a heteroreceptor with c-erbB-2.
(19) The synoviocyte growth inhibitor according to the above (17), wherein the antagonists of c-erbB-2 or receptors forming heteroreceptors with c-erbB-2 are neutralizing antibodies recognizing c-erbB-2 or receptors forming heteroreceptors with c-erbB-2, fragments of said antibodies, or derivatives thereof.
(20) The synoviocyte growth inhibitor according to the above (18), wherein the substances which bind to an erbB ligand and inhibit the binding of the erbB ligand to c-erbB-2 or a receptor forming a heteroreceptor with c-erbB-2 are neutralizing antibodies recognizing erbB ligands, fragments of said antibodies, or derivatives thereof.
(21) The synoviocyte growth inhibitor according to the above (15), wherein the substances which inhibit the signal transduction after the binding of an erbB ligand to c-erbB-2 are substances which inhibit the tyrosine phosphorylation of c-erbB-2 or substances which inhibit the interaction between phosphorylated c-erbB-2 and an intracellular signaling molecule.
(22) The synoviocyte growth inhibitor according to the above (21), wherein the substances which inhibit the tyrosine phosphorylation of c-erbB-2 are tyrosine kinase inhibitors.
(23) The synoviocyte growth inhibitor according to the above (21), wherein the substances which inhibit the interaction between phosphorylated c-erbB-2 and an intracellular signaling molecule are antibodies to the intracellular domain of c-erbB-2, fragments of said antibodies, or derivatives thereof.
(24) The synoviocyte growth inhibitor according to the above (21), wherein the substance which inhibits the expression of c-erbB-2 is selected from the group consisting of antisense DNAs, antisense oligonucleotides, triple helix-forming oligonucleotides and ribozymes for the c-erbB-2 gene.
(25) A diagnostic agent for rheumatoid arthritis comprising, as an active ingredient, a substance which specifically binds to c-erbB-2 or to an mRNA or cDNA for c-erbB-2.
(26) The diagnostic agent for rheumatoid arthritis according to the above (25), wherein the substance which binds to c-erbB-2 or to an mRNA or cDNA for c-erbB-2 is at least one member selected from the group consisting of antibodies recognizing c-erbB-2, DNAs of c-erbB-2 gene, fragments of said DNAs, and oligonucleotides consisting of 10 to 50 nucleotides identical to the nucleotide sequences of said DNAs or complementary strands thereof.
(27) A diagnostic method for rheumatoid arthritis which comprises detecting or determining c-erbB-2 in synoviocytes by using the diagnostic agent according to the above (25) or (26).

### 1. Therapeutic Agent for Rheumatoid Arthritis and Synoviocyte Growth suppressor

The therapeutic agent for rheumatoid arthritis or synoviocyte growth suppressor of the present invention may be any of the substances which inhibit the biological activities of c-erbB-2. The substances which inhibit the biological activities of c-erbB-2 include substances which inhibit the signal transduction mediated by c-erbB-2, substances which suppress the expression of c-erbB-2, and the like.

The substances which inhibit the signal transduction mediated by c-erbB-2 include substances which inhibit the binding of an erbB ligand to c-erbB-2, substances which inhibit the binding of an erbB ligand to a receptor comprising c-erbB-2, substances which inhibit the dimerization of c-erbB-2, substances which inhibit the signal transduction after the binding of an erbB ligand to c-erbB-2, and the like.

The erbB ligand may be any ligand that has the ability to cause activation of c-erbB-2. Suitable erbB ligands include ligands which cause activation of c-erbB-2 by binding to receptors forming heteroreceptors, preferably heterodimer receptors, with c-erbB-2, e.g. receptors of the erbB family such as EGFR, ligands which cause activation of c-erbB-2 by directly binding to c-erbB-2, and the like. Examples of such ligands are EGF and TGFα.

Preferred substances which inhibit the signal transduction mediated by c-erbB-2 are neutralizing antibodies recognizing c-erbB-2 (hereinafter referred to as anti-c-erbB-2 neutralizing antibodies), fragments of said antibodies, and derivatives thereof.

The antibodies include polyclonal antibodies and monoclonal antibodies.

The monoclonal antibodies include antibodies produced by hybridomas, humanized antibodies, human antibodies, and the like.

The antibody fragments include Fab (fragment of antigen binding), Fab', F(ab')₂, single chain Fv (hereinafter referred to as scFv), disulfide stabilized Fv (dsFv), peptides containing complementarity determining region (hereinafter referred to as CDR), and the like.

The "derivatives of antibodies or antibody fragments" refers to antibodies in which a radioisotope, a protein, a low molecular compound or the like is bound to antibodies produced by hybridomas, humanized antibodies, human antibodies, or fragments of said antibodies.

Examples of the anti-c-erbB-2 neutralizing antibodies include the antibodies reported in Mol. Cell. Biol., 9, 1165-1172 (1989); Int. J. Can., 53, 401-408 (1993); Am. J. Resp. Cell. Mol. Biol., 9, 448-454 (1993); etc., and Herceptin [Clin. Ther., 21, 309-318 (1999)].

The receptor comprising c-erbB-2 may be any receptor comprising c-erbB-2, e.g., c-erbB-2 alone, a c-erbB-2 homodimer or a c-erbB-2 heterodimer. A c-erbB-2 heterodimer receptor is comprised by c-erbB-2 and a member of the erbB family, e.g., EGFR. A receptor forming a heterodimer receptor with c-erbB-2 is hereinafter referred to as a heteroreceptor.

The substances which inhibit the binding of an erbB ligand to c-erbB-2 and the substances which inhibit the binding of an erbB ligand to a receptor comprising c-erbB-2 include antagonists of c-erbB-2 or heteroreceptors, substances which bind to an erbB ligand and inhibit the binding of the erbB ligand to c-erbB-2 or a heteroreceptor, and the like.

The antagonists of c-erbB-2 or heteroreceptors include neutralizing antibodies recognizing c-erbB-2 or heteroreceptors, fragments of said antibodies, derivatives thereof, substances which bind to c-erbB-2 or a heteroreceptor competitively with an erbB ligand but which are incapable of activating the receptor, and the like. The antibody fragments and the derivatives of antibodies or antibody fragments have the same meanings as given above.

Examples of such antagonists include an EGF derivative which binds to EGFR competitively with EGF but which is incapable of activating the heteroreceptor [Pathol. Res. Pract., 192, 761-767 (1996)], potato caroboxypeptidase inhibitor which is considered to be similar to EGF in higher-order structure [J. Biol. Chem., 273, 12370-12377 (1998)], a TGF-α partial peptide which competes with TGF-α [Biochem. Biophys. Res. Commun., 129, 226-232 (1985)] and methylpheophorbid [Oncol. Res., 4, 193-200 (1992)].

The substances which bind to an erbB ligand and inhibit the binding of the erbB ligand to c-erbB-2 or a heteroreceptor include neutralizing antibodies recognizing erbB ligands such as EGF and TGF-α, fragments of said antibodies, derivatives thereof, secretory receptors comprising only the extracellular domain of heteroreceptors such as EGFR, and the like. The antibody fragments and the derivatives of antibodies or antibody fragments have the same meanings as given above.

Examples of such substances include secretory EGFR comprising only the extracellular domain of EGFR [Mol. Cell. Biol., 12, 491-498 (1992); Mol. Cell. Biol., 12, 883-893 (1992)].

The substances which inhibit the dimerization of c-erbB-2 include antibodies to c-erbB-2 which are incapable of activating c-erbB-2, fragments of such antibodies, derivatives thereof, c-erbB-2 mutants in which amino acids after the 341st amino acid in the amino acid sequence of c-erbB-2 responsible for the dimerization of c-erbB-2 are deleted, c-erbB-2 dominant negative mutants which are not activated by the binding of an erbB ligand to an erbB receptor, and the like. The antibody fragments and the derivatives of antibodies or antibody fragments have the same meanings as given above.

Examples of such substances include herstatin, a c-erbB-2 splicing variant which has the c-erbB-2-binding ability and in which amino acids after the 341st amino acid in the amino acid sequence of c-erbB-2 are deleted [Proc. Natl. Acad. Sci. USA., 96, 10869-10874 (1999)], c-erbB-2 mutants in which amino acids in the c-erbB-2 intracellular domain are deleted [Oncogene, 18, 3481-3490 (1999); J. Biol. Chem., 274, 8900-8909 (1999)] and c-erbB-2 mutants mutated in amino acids in the c-erbB-2 kinase domain [Oncogene, 9, 1507-1514 (1994)].

The substances which inhibit the signal transduction after the binding of an erbB ligand to c-erbB-2 include substances which inhibit the tyrosine phosphorylation of c-erbB-2 and substances which inhibit the interaction between phosphorylated c-erbB-2 and an intracellular signaling molecule.

The substances which inhibit the tyrosine phosphorylation of c-erbB-2 may be any substances that inhibit tyrosine phosphorylation. For example, they include tyrosine kinase inhibitors such as genistein [Int. J. Oncol., 15, 525-533 (1999)], emodin [Cancer Res., 55, 3890-3896 (1995)] and geldanamycin [J. Med. Chem., 38, 3806-3812 (1995)], tyrosine phosphatase, antibodies to the region of c-erbB-2 containing tyrosine to be phosphorylated, fragments of said antibodies, and derivatives of said antibodies or antibody fragments. The antibody fragments and the derivatives of antibodies or antibody fragments have the same meanings as given above.

Preferred substances which inhibit the tyrosine phosphorylation of c-erbB-2 are those which are specific to c-erbB-2, because the phosphorylation of intracellular protein by tyrosine kinase is concerned with various kinds of signal transduction in cells. Examples of c-erbB-2-specific substances which inhibit tyrosine phosphorylation include 6 or 7-acrylamide-4-anilinoquinazoline [Proc. Natl. Acad. Sci. USA., 95, 12022-12027 (1998)], 3-phenylindolin-2-one [J. Med. Chem., 41, 2588-2601 (1998)] and tylophostin [J. Biol. Chem., 268, 11134-11142 (1993)].

The substances which inhibit the interaction between phosphorylated c-erbB-2 and an intracellular signaling molecule include antibodies to the intracellular domain of c-erbB-2, fragments of said antibodies, derivatives thereof, and the like. The antibody fragments and the derivatives of antibodies or antibody fragments have the same meanings as given above.

The substances which suppress the expression of c-erbB-2 include antisense DNAs, antisense oligonucleotides, triple helix-forming oligonucleotides and ribozymes for the c-erbB-2 gene, compounds specifically inhibiting transcription or translation, and the like.

The "expression of c-erbB-2" includes all steps of the process of expression such as the transcription of the c-erbB-2 gene to mRNA and the translation from the mRNA into the c-erbB-2 protein.

The methods for suppressing the expression of c-erbB-2 include suppression of translation from mRNA into the c-erbB-2 protein by administration of an antisense DNA or antisense oligonucleotide for the c-erbB-2 gene or by administration of an antisense RNA expression vector [Br. J. Can., 70, 819-125 (1994); Biochem. Biophys. Res. Commun., 200, 661-667 (1994); Int. J. Can., 72, 631-636 (1997)], inhibition of transcription by administration of a triple helix-forming oligonucleotide binding to the c-erbB-2 gene promoter [Biochemistry, 38, 619-628 (1999); Can. Res., 56, 515-522 (1996); Gene, 149, 123-126 (1994)], degradation of the c-erbB-2 mRNA by administration of a ribozyme [J. Biol. Chem., 272, 29482-29486 (1997)], inhibition of transcription by administration of a compound specifically inhibiting the transcription of the c-erbB-2 gene to mRNA [Brit. J. Can., 71, 753-757 (1995)], and the like.

The compounds specifically inhibiting the transcription can be screened by the reporter assay [Takaaki Tamura (ed.), Tensha Inshi Kenkyuho (Methods for Research on Transcription Factors), Yodosha (1993); Latchman DS, Transcription Factors: A Practical Approach, IRL Press (1993)]. That is, screening for the compounds specifically inhibiting the transcription can be carried out by isolating the promoter region of c-erbB-2 [Gene, 136, 361-364 (1993); J. Biol. Chem., 265, 4389-4393 (1990); Mol. Cell. Biol., 7, 2597-2601 (1987)] from genomic DNA prepared from human cells by means of PCR [Dieffenbach CW & Dveksler GS, PCR Primer: A Laboratory Manual, Cold Spring Harbor Laboratory (1995)] or the like, and then expressing in animal cells a vector comprising a unit in which a reporter gene whose expression level can be easily determined is inserted downstream of the promoter region. Suitable reporter genes include luciferase gene and chloramphenicol acetyltransferase gene.

Test compounds are added to the above cells in which the reporter gene is to be expressed, and the level of the transcription of the reporter gene is determined and compared with that determined without the addition of a test compound. Test compounds which lower the transcription level are selected as the compounds specifically inhibiting the transcription.

The substances which inhibit the biological activities of c-erbB-2 of the present invention have synoviocyte growth-inhibiting activity and can be used for the treatment of rheumatoid arthritis.

When the substance which inhibits the biological activities of c-erbB-2 of the present invention is a protein, this protein can be expressed in the synovial tissue of a patient by preparing a vector for gene therapy carrying a gene encoding the protein and administering the vector to the patient, whereby the inhibition of synoviocyte growth or the treatment of rheumatoid arthritis can be effected.

### 2. Pharmaceutical Preparation for the Treatment of Rheumatoid Arthritis

The pharmaceutical preparation of the present invention for the treatment of rheumatoid arthritis and the inhibition of synoviocyte growth comprising a substance which inhibits the biological activities of c-erbB-2 as an active ingredient may comprise, as an active ingredient, the substance alone or as a mixture with any other active ingredient for another therapeutic purpose. The pharmaceutical preparation can be produced according to any of the methods well known in the technical field of pharmaceutics by mixing an active ingredient with one or more pharmaceutically acceptable carriers.

It is desirable to administer the preparation by the route that is most effective for the treatment. Suitable administration routes include oral administration and non-oral administration such as intravenous administration.

The preparation is administered in the form of tablets, powders, granules, syrup, injection, or the like.

Liquid preparations suitable for oral administration such as syrup can be prepared using water, sugars (e.g., sucrose, sorbitol and fructose), glycols (e.g., polyethylene glycol and propylene glycol), oils (e.g., sesame oil, olive oil and soybean oil), antiseptics (e.g., p-hydroxybenzoates), flavors (e.g., strawberry flavor and peppermint), and the like. Tablets, powders, granules, etc. can be prepared using excipients (e.g., lactose, glucose, sucrose and mannitol), disintegrators (e.g., starch and sodium alginate), lubricants (e.g., magnesium stearate and talc), binders (e.g., polyvinyl alcohol, hydroxypropyl cellulose and gelatin), surfactants (e.g., fatty acid esters), plasticizers (e.g., glycerin), and the like.

Preparations suitable for non-oral administration preferably comprise a sterilized aqueous preparation containing an active substance which is isotonic to the recipient's blood. In the case of an injection, for example, a solution for injection is prepared using a carrier comprising a salt solution, a glucose solution, or a mixture of salt water and a glucose solution. The non-oral preparations may also comprise one or more auxiliary components selected from the diluents, antiseptics, flavors, excipients, disintegrators, lubricants, binders, surfactants, plasticizers, etc. mentioned in the above description of oral preparations.

The dose and the administration schedule of the pharmaceutical preparation of the present invention will vary depending upon the administration route, the age and body weight of a patient, and the symptom and degree of the disease to be treated. Usually, in the case of oral administration, the active ingredient is administered in a dose of 0.01 mg to 1 g, preferably 0.05 to 50 mg, per adult once to several times per day. In the case of non-oral administration such as intravenous administration, the active ingredient is administered in a dose of 0.001 to 100 mg, preferably 0.01 to 10 mg, per adult once to several times per day. However, the dose and the administration schedule may vary depending upon various conditions as given above.

### 3. Diagnostic Agent for Rheumatoid Arthritis Patients

The diagnostic agents for rheumatoid arthritis include substances specifically binding to a protein which is expressed at low levels in synoviocytes of normal persons but which is expressed at high levels in synoviocytes of rheumatoid arthritis patients, and substances specifically binding to an mRNA or cDNA of such a protein. The expression level of such a protein in a sample can be measured by conducting the detection or determination of the protein or the mRNA for the protein using the above substances.

An example of the protein which is expressed at low levels in synoviocytes of normal persons but which is expressed at high levels in synoviocytes of rheumatoid arthritis patients is c-erbB-2. Therefore, substances useful in the determination or detection of c-erbB-2 in synoviocytes are useful as diagnostic agents for rheumatoid arthritis. The present invention relates to a diagnostic agent for rheumatoid arthritis comprising, as an active ingredient, a substance capable of determinating or detecting of c-erbB-2 in synoviocytes, and to a diagnostic method for rheumatoid arthritis using this diagnostic agent. The substances capable of determinating or detecting c-erbB-2 include anti-c-erbB-2 antibodies, fragments of said antibodies, derivatives thereof, DNAs of c-erbB-2 gene capable of determinating or detecting mRNA for c-erbB-2, fragments of said DNAs, oligonucleotides consisting of 10 to 50 nucleotides identical to the nucleotide sequences of complementary strands of said DNAs or DNA fragments, and the like.

### 4. Diagnostic Method for Rheumatoid Arthritis

Samples suitable for use in the diagnosis of rheumatoid arthritis according to the present invention include biopsy samples of synovial tissues collected from the joints of subjects by surgical or other methods. Synoviocytes isolated from the synovial tissues collected as above and then cultured by the method described in Biochem. Biophys. Res. Com., 210, 1066-75 (1995) may also be used as samples, according to need.

### (1) Diagnostic Method for Rheumatoid Arthritis in Which the Expression Level of c-erbB-2 is Immunologically Measured

Diagnosis of rheumatoid arthritis according to the method of the present invention can be carried out, for example, by immunologically detecting or determining c-erbB-2 present in the synovial tissue or synoviocytes of a subject using an antibody recognizing c-erbB-2, a fragment of said antibody, or a derivative thereof as described below, and comparing the result with the expression level of c-erbB-2 in normal synovial tissues or synoviocytes.

The methods of immunologically detecting c-erbB-2 present in synovial tissues or synoviocytes using an antibody recognizing c-erbB-2, a fragment of said antibody, or a derivative thereof include immunohistochemical staining such as immunohisto staining or immunocyte staining, enzyme immunoassay (EIA) and radioimmunoassay (RIA) such as flow cytometry, Western blotting and sandwich ELISA, and the like. These methods can be carried out based on the descriptions in the literature [Sakuji Toyama and Tamie Ando (eds.), A Manual of Experiments on Monoclonal Antibodies, Kodansha Scientific (1987); Zoku Seikagaku Jikken Koza 5 (Lectures on Experiments in Biochemistry, second series, 5), Menekiseikagaku Kenkyuho (Methods for Immunobiochemical Research), Tokyo Kagaku Dojin (1986); Goding JW, Monoclonal Antibodies: Principles and Practice, Third edition, Academic Press (1996): Harlow E & Lane D, Antibodies: A Laboratory Manual, Cold Spring Harbor Laboratory, (1988)].

According to immunohistochemical staining, a synovial tissue section taken from the joint of a subject or synoviocytes isolated from the section are fixed and subjected to reaction with an antibody or antibody fragment recognizing c-erbB-2 and then to reaction with an anti-immunoglobulin antibody or antibody fragment labeled with a fluorescent substance, an enzyme, biotin, gold colloid, a radioactive substance or the like, followed by, if necessary after visualization of the labeled antibody, microscopic observation to detect c-erbB-2 in the tissue or cells. In the case of fluorescence labeling, fluorescein isothiocyanate (FITC), tetramethylrhodamine isothiocyanate or the like is used for labeling, and detection is carried out by observation under a fluorescence microscope. In the case of enzyme labeling, peroxidase, alkaline phosphatase or the like is used, and after coloring reaction with addition of a substrate coloring in the presence of an enzyme, detection is carried out by observation under a optional microscope. In the case of biotinylation, reaction with avidin labeled with an enzyme such as peroxidase is carried out and then a procedure similar to that in enzyme labeling is carried out. In the case of gold colloid labeling, detection is carried out by observation under an electron microscope. In the case of radiolabeling, ¹²⁵I or the like is used for labeling, and after the tissue section or cells are coated with a photosensitive emulsion, detection is carried out by optional microscopic observation of silver particles deposited with radiation.

According to flow cytometry, synoviocytes isolated from synovial tissue taken from the joint of a subject are subjected to reaction with an antibody recognizing c-erbB-2 and then to reaction with an anti-immunoglobulin antibody or antibody fragment labeled with a fluorescent substance such as fluorescin isothiocyanate or phycoerythrin, followed by measurement of a fluorescent pigment with a flow cytometer to detect the expression of c-erbB-2 in the cells.

According to Western blotting, synovial tissue taken from the joint of a subject, synoviocytes isolated from the tissue, or a disrupted tissue or cell suspension prepared therefrom is fractionated by SDS-polyacrylamide gel electrophoresis, followed by blotting on PVDF membrane or nitrocellulose membrane. The membrane is subjected to reaction with an antibody or antibody fragment recognizing c-erbB-2 and then to reaction with an anti-immunoglobulin antibody or antibody fragment labeled with an enzyme (e.g., peroxidase or alkaline phosphatase) or a radioactive substance (e.g., ¹²⁵I), followed by detection of the band of c-erbB-2. In the case of enzyme labeling, detection is carried out by visualization of the band of c-erbB-2 by reaction caused by addition of a substrate coloring in the presence of an enzyme, or by autoradiography using X ray film with addition of a substrate coloring in the presence of an enzyme. In the case of radiolabeling, detection is carried out by autoradiography using X ray film.

Sandwich ELISA, which is a kind of enzyme immunoassay, is carried out using two kinds of monoclonal antibodies recognizing c-erbB-2 which have different antigen recognition sites. Prior to reaction, one monoclonal antibody or antibody fragment is adsorbed on a plate and the other monoclonal antibody or antibody fragment is labeled with an enzyme such as peroxidase or alkaline phosphatase. A disrupted cell suspension is prepared from synovial tissue taken from the joint of a subject or synoviocytes isolated from the tissue and is used as a test sample. The antibody-adsorbed plate is subjected to reaction with the test sample and then to reaction with the enzyme-labeled c-erbB-2 monoclonal antibody or antibody fragment. After coloring reaction caused by addition of a substrate coloring in the presence of the enzyme, the color intensity is measured with a spectrophotometer, whereby the detection or determination of c-erbB-2 in the sample can be effected.

In radioimmunoassay, a procedure similar to that in enzyme immunoassay is carried out using an antibody labeled with a radioactive substance such as ¹²⁵I instead of an enzyme. Then, radioactivity is measured with a scintillation counter, whereby the detection or determination of c-erbB-2 in the sample can be effected.

Enzyme immunoassay and radioimmunoassay can be carried out by competitive assay, besides the above-described sandwich assay. In competitive assay, c-erbB-2 preparation, instead of antibody, is labeled and a certain amount of the labeled c-erbB-2 preparation and a test sample are subjected to reaction with an antibody or antibody fragment recognizing c-erbB-2 which has been solid-phased on a plate. By measuring the enzyme activity or radioactivity on the plate, the detection or determination of c-erbB-2 in the sample can be effected.

### (2) Diagnostic Method in Which the Expression Level of c-erbB-2 is Measured at mRNA Level

It is highly probable that the elevation in the expression level of c-erbB-2 in synoviocytes occurs at mRNA level. Therefore, diagnosis of rheumatoid arthritis can also be made by measuring the amount of c-erbB-2 mRNA, not c-erbB-2 protein. Specifically, diagnosis can be made by preparing RNA from synovial tissue taken from the joint of a subject or synoviocytes isolated from the tissue, measuring the mRNA level by c-erbB-2 mRNA-specific RT-PCR [Dieffenbach CW & Dveksler GS, PCR Primer: A Laboratory Manual, Cold Spring Harbor Laboratory (1995)] or Northern blot hybridization, and comparing the mRNA level with that of normal synoviocytes. In RT-PCR, oligonucleotides consisting of 10 to 50 nucleotides identical to the nucleotide sequence of c-erbB-2 cDNA (GenBank accession No. X03363) or complementary strands thereof, which can be prepared by using a DNA synthesizer, can be used as primers. Probes useful in Northern blot hybridization include c-erbB-2 cDNAs and fragments thereof labeled with a radioisotope (e.g., ³²P), digoxigenin or the like. The c-erbB-2 cDNAs and fragments thereof can be prepared by RT-PCR using RNA prepared from a cell line reported to have been expressing c-erbB-2, e.g., MKN-7 [Nature, 319, 230-234 (1986)]. Northern blot hybridization and preparation of RNA can be carried out according to the methods described in a laboratory manual [Sambrook, J., et al., Molecular Cloning: A Laboratory Manual, Second Edition, Cold Spring Harbor Laboratory (1989)].

### Brief Description of the Drawings

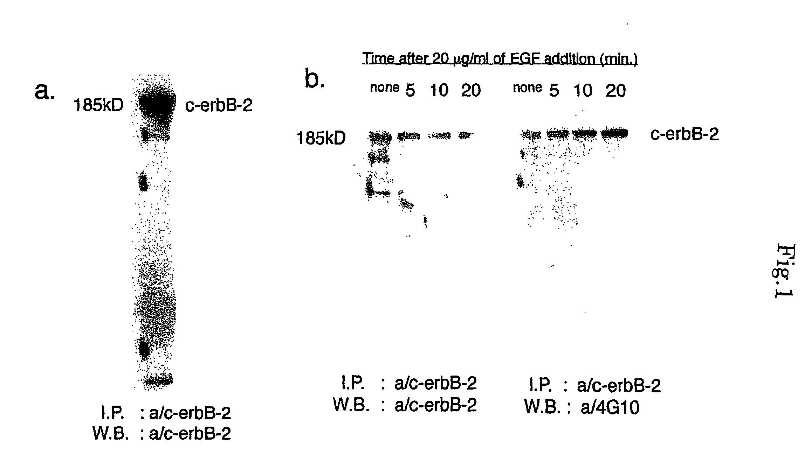

Fig. 1a: Detection of c-erbB-2 in synoviocytes derived from a rheumatoid arthritis patient. The figure shows the result of Western blotting carried out on the synoviocytes using an anti-c-erbB-2 antibody.
Fig. 1b: Tyrosine phosphorylation of c-erbB-2 in the synoviocytes caused by addition of EGF. The figure shows the result of Western blotting carried out on immune precipitates formed by reaction of the synoviocytes with an anti-c-erbB-2 antibody intermittently after EGF addition. The left four lanes show the results of detection with an anti-c-erbB-2 antibody, and the right four lanes show the results of detection with an anti-phosphorylated tyrosine antibody [none: before EGF addition, numbers: time after EGF addition (minute)].

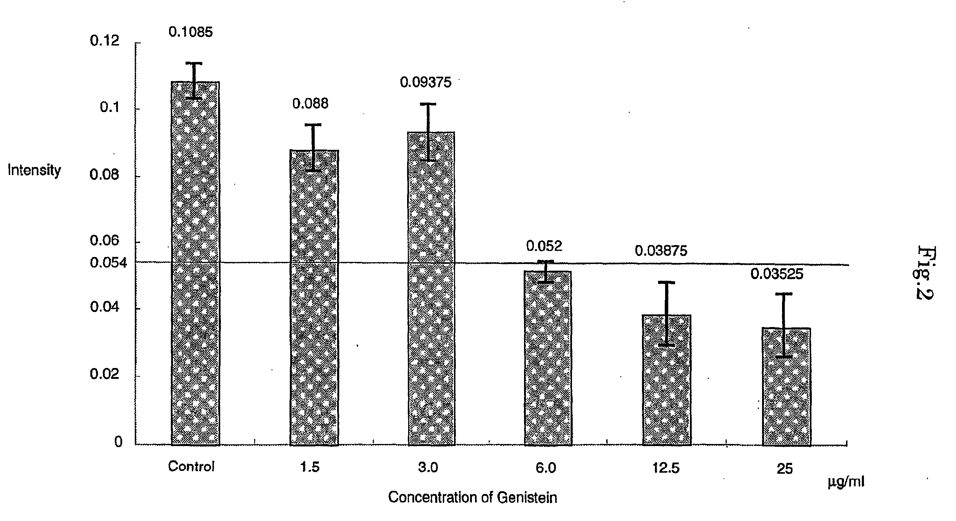

Fig. 2: Inhibition of the growth of the synoviocytes by addition of genistein. The numbers on the abscissa of the bar graph indicate the concentration of genistein added (µg/ml), and the numbers on the ordinate indicate the average absorbance at 590 nm, showing the degree of cell growth. The average absorbance at each concentration is numerically indicated above each bar and the standard error is indicated by a vertical line.

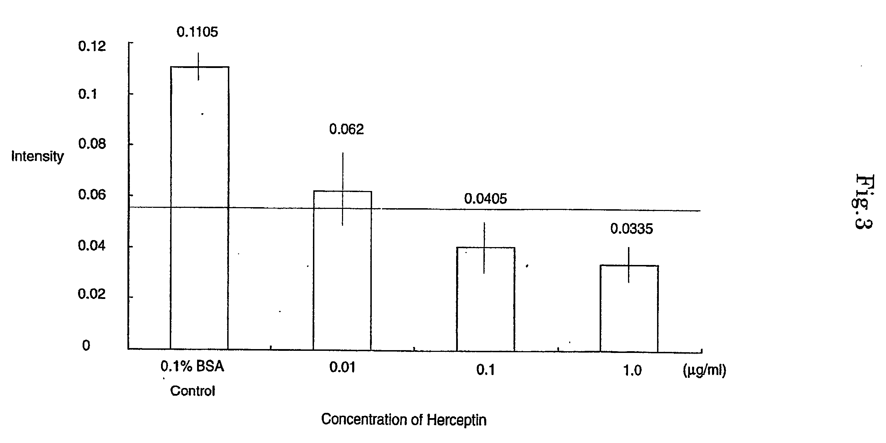

Fig. 3: Inhibition of the growth of the synoviocytes by addition of Herceptin. The numbers on the abscissa indicate the concentration of Herceptin added (µg/ml), and the numbers on the ordinate indicate the average absorbance at 590 nm, showing the degree of cell growth. The average absorbance at each concentration is numerically indicated above each bar and the standard error is indicated by a vertical line.

### Best Modes for Carrying Out the Invention

### Example 1 Expression of the c-erbB-2 Gene in Synoviocytes of a Rheumatoid Arthritis Patient

### (1) Preparation of Synoviocytes

Synoviocytes were prepared from the diseased part of a rheumatoid arthritis patient according to the method described in Biochem. Biophys. Res. Com., 210, 1066-75 (1995). That is, synoviocytes obtained from a rheumatoid arthritis patient by surgery were minced, followed by enzyme treatment in RPMI1640 medium containing collagenase (4 mg/ml) at 37°C for 4 hours to loosen the cells. The obtained cells were cultured in a growth medium (RPMI1640 containing 10% fetal calf serum) in a humidified CO₂ incubator (5% CO₂), with replacement of the growth medium at intervals of 3 to 4 days. When the cells grew to be subconfluent, the cells were subcultured at the ratio of 1:2 to 1:4 (that is, the cells were collected, transferred into other culture flasks in 1/2 to 1/4 portions and cultured in a fresh growth medium). Subculturing was repeated for 7 to 15 passages and the resulting cells were used in the following experiments.

### (2) Detection of the Expression of the c-erbB-2 Protein in the Synoviocytes by Western Blotting

The subconfluent synoviocytes were washed with ice-cooled phosphate buffer (PBS) and then lysed in a buffer [20 mM Tris-hydrochloride buffer (pH 7.5), 1 mM EDTA, 50 mM sodium fluoride, 50 mM β-glycerophosphate, 0.05 mM Na₃VO₄, 10 µg/ml leupeptin, 10 µg/ml aprotinin, 100 mM PMSF] containing 1% Nonidet P-40 at 4°C for one hour, followed by centrifugation. The obtained supernatant (hereinafter referred to as the cell lysate) was subjected to the following immune precipitation.

To the cell lysate was added a mouse anti-c-erbB-2 antibody (Calbiochem) and the mixture was incubated overnight at 4°C to form an immune complex. To the immune complex was added 30 µl of protein G-Sepharose (50% slurry, Zymed Laboratories), followed by incubation at 4°C for 30 minutes to bind the immune complex.

Protein G-Sepharose was recovered and washed five times with Tris buffer (pH 7.6) containing 0.1% Tween 20, followed by heating in 20 ml of a buffer for SDS-PAGE sample [62.5 mM Tris-hydrochloride (pH 6.8), 10% glycerol, 2.3% SDS, 5% 2-mercaptoethanol] at 90°C for 5 minutes to dissolve the immune complex in the buffer and then to obtain an immune precipitate. The obtained immune precipitate was subjected to Western blotting to detect c-erbB-2.

The protein was separated by SDS-PAGE of the immune precipitate, followed by blotting on PVDF filter to transfer the protein. After blocking of the non-specific binding on the filter using Tris buffer containing 5% skim milk, the filter was subjected to reaction with a mouse anti-c-erbB-2 antibody (Calbiochem). Subsequently, the filter was subjected to reaction with a peroxidase-labeled goat anti-mouse IgG antibody (Dako) as a secondary antibody, followed by detection of a band using detection system ECL (Amersham Pharmacia Biotech) based on chemiluminescence.

The result of Western blotting is shown in Fig. 1a. A distinct band of 185 kDa reacting with the c-erbB-2 antibody was detected. This molecular weight agreed with the reported molecular weight of c-erbB-2 on cells. Therefore, it was demonstrated that c-erbB-2 was expressed in a large quantity in the synoviocytes of the rheumatoid arthritis patient.

### (3) Phosphorylation of c-erbB-2 by EGF

When c-erbB-2 and EGFR coexist on cells, addition of EGF causes heterodimerization and activation of c-erbB-2 and EGFR, thus leading to phosphorylation of the tyrosine residue in the intracellular domain of c-erbB-2. EGF was added to the synoviocytes prepared in (1) and the degree of phosphorylation of c-erbB-2 was intermittently examined in the following manner.

The synoviocytes were collected 5, 10 and 20 minutes after the addition of 20 µg/ml EGF and subjected to immune precipitation using an anti-c-erbB-2 antibody in the same manner as in (2). After each of the obtained immune precipitates was subjected to SDS-PAGE (7.5% acrylamide), Western blotting was carried out in the same manner as in (2) using, as a primary antibody, an anti-c-erbB-2 antibody or an anti-phosphorylated tyrosine antibody 4G10 (Upstate Biotechnology) to detect a band. The results are shown in Fig. 1b.

There was observed little change in the amount of the c-erbB-2 protein reacting with the anti-c-erbB-2 antibody, whereas the amount of phosphorylated c-erbB-2 reacting with the anti-phosphorylated tyrosine antibody increased with the passage of time. Thus, it was confirmed that c-erbB-2 in the synoviocytes was activated and phosphorylated by EGF. The band of tyrosine phosphorylated c-erbB-2 was detected also in the synoviocytes without EGF addition. This suggests that synoviocytes of a rheumatoid arthritis patient themselves secrete ligands such as EGF to somewhat activate c-erbB-2 in themselves.

### (4) Detection of c-erbB-2 in Synovial Tissue of a Rheumatoid Arthritis Patient by Immunostaining of Tissue

Synovial tissues were obtained from a rheumatoid arthritis patient and, as a control, the ankle of a normal rat by surgery. The obtained tissue was fixed in a buffer containing 4% paraformaldehyde and then embedded in paraffin to prepare a section 4 µm thick. In order to raise the immune reactivity, the section was treated with 0.1% trypsin (Zymed Laboratories) on a slide at 37°C for 10 minutes. After washing, the section was incubated in methanol containing 0.3% hydrogen peroxide at room temperature for 30 minutes to inhibit the inherent peroxidase activity. The section was then treated with 3% normal goat serum (Vector Laboratories Inc.)/PBS at room temperature for one hour to block the non-specific binding part. Subsequently, incubation was carried out in a humidified chamber with 2.5 mg/ml mouse anti-c-erbB-2 monoclonal antibody (Calbiochem) as a primary antibody at room temperature for 24 hours and then with biotinylated goat anti-mouse IgG antibody (Vector Laboratories Inc.) as a secondary antibody at room temperature for one hour. Further, incubation was carried out with an avidin-biotin-peroxidase complex reaction reagent containing peroxidase-labeled avidin at room temperature for one hour. After thorough washing, incubation was carried out with DAB (3,3'-diaminobenzidine tetrahydrochloride, Wako Pure Chemical Industries, Ltd.) solution at room temperature for 10 minutes. Then, brown coloring reaction was caused by addition of DAB solution containing 3% hydrogen peroxide, and prior to the change of brown color to black, the reaction was stopped by washing in a buffer. Separately, tissue sections prepared in the same manner as above were subjected to cell nucleus staining using Methyl Green (Wako Pure Chemical Industries, Ltd.). These stained tissue sections were put on glass slides using Canada balsam (Wako Pure Chemical Industries, Ltd.) and observed under a optional microscope.

As a result, the synovial tissue from the rheumatoid arthritis patient was observed to have been strongly stained with the anti-c-erbB-2 antibody compared with the synovial tissue from the normal rat ankle. By nuclear staining, a lot of inflammatory exudate cells were observed with the synovial tissue from the rheumatoid arthritis patient.

### Example 2 Inhibition of Growth of Synoviocytes by Inhibition of Phosphorylation of c-erbB-2

Participation of c-erbB-2 expressed in synoviocytes in the growth of synoviocytes was clarified by inhibition of phosphorylation of c-erbB-2 in the following manner. The growth of synoviocytes was measured by the assay using MTT [3,(4,5-dimethylthiazol-2-yl)2,5-diphenyl-tetrazolium bromide] [J. Immunol. Methods, 65, 55-63 (1983)].

### (1) Growth Inhibition by Tyrosine Kinase Inhibitor Genistein

Synoviocytes derived from a rheumatoid arthritis patient which were prepared in the same manner as in Example 1 were seeded on a 96-well microplate in an amount of 5 x 10³ cells/well and precultured in the growth medium at 37°C overnight. After the cells that adhered to the plate were washed, culturing was carried out using 200 µl each of growth media respectively containing tyrosine kinase inhibitor genistein at concentrations of 1.5, 3, 6, 12.5 and 25 µg/ml, and as a control, 200 µl of RPMI1640 [supplemented with 0.1% bovine serum albumin (BSA), serum free] containing no genistein at 37°C for 7 days, during which the medium was replaced with a fresh one every two days. On the last day of culturing, 10 µl of 5 mg/ml MTT solution was added, followed by culturing for 2 hours. Then, 100 µl of dimethyl sulfoxide was added to dissolve the formazan complex and the absorbance at the wavelength of 590 nm was measured with 96-well multiscanner Immno-Mini (Japan Inter Med) as the degree of cell growth. This experiment was carried out four times in total, and the average value and the standard error (SE) were obtained.

The results are shown in Fig. 2. The growth of synoviocytes was inhibited in a manner dependent on the genistein concentration, which suggested the participation of tyrosine phosphorylation of c-erbB-2 in the growth of synoviocytes.

### (2) Growth Inhibition by Anti-c-erbB-2 Antibody

Genistein is not a drug inhibiting only the autophosphorylation of c-erbB-2 but a drug inhibiting tyrosine kinase in general. Therefore, the action of Herception (Genentech Inc.), which is an anti-c-erbB-2 monoclonal antibody considered to specifically inhibit the activation of c-erbB-2, on the growth of synoviocytes was examined in the same manner as in (1). Herceptin was added at concentrations of 0.01, 0.1 and 1 µg/ml, respectively, and as a control, RPMI1640 (supplemented with 0.1% BSA, serum free) containing no Herceptin was used. The results are shown in Fig. 3. The growth of synoviocytes was inhibited in a manner dependent on the Herceptin concentration, which indicated that the growth of synoviocytes was dependent on c-erbB-2.

The results of (1) and (2) revealed that drugs inhibiting activation or tyrosine phosphorylation of c-erbB-2, such as anti-c-erbB-2 antibodies and tyrosine kinase inhibitors, can inhibit the growth of synoviocytes.

### Industrial Applicability

The present invention provides a therapeutic agent for rheumatoid arthritis which inhibits the growth of synoviocytes mediated by c-erbB-2.

## Claims

1. A therapeutic agent for rheumatoid arthritis comprising, as an active ingredient, a substance which inhibits the biological activities of c-erbB-2.

2. The therapeutic agent for rheumatoid arthritis according to Claim 1, wherein the substance which inhibits the biological activities of c-erbB-2 is a substance which inhibits the signal transduction mediated by c-erbB-2 or a substance which suppresses the expression of c-erbB-2.

3. The therapeutic agent for rheumatoid arthritis according to Claim 2, wherein the substance which inhibits the signal transduction mediated by c-erbB-2 is at least one member selected from the group consisting of substances which inhibit the binding of an erbB ligand to c-erbB-2, substances which inhibit the binding of a c-erbB-2 ligand to a receptor comprising c-erbB-2, substances which inhibit the dimerization of c-erbB-2, and substances which inhibit the signal transduction after the binding of an erbB ligand to c-erbB-2.

4. The therapeutic agent for rheumatoid arthritis according to Claim 2, wherein the substance which inhibits the signal transduction mediated by c-erbB-2 is at least one member selected from the group consisting of neutralizing antibodies recognizing c-erbB-2, fragments of said antibodies, and derivatives thereof.

5. The therapeutic agent for rheumatoid arthritis according to Claim 3, wherein the substances which inhibit the binding of an erbB ligand to c-erbB-2 or the substances which inhibit the binding of an erbB ligand to a receptor comprising c-erbB-2 are c-erbB-2 antagonists or antagonists of receptors forming heteroreceptors with c-erbB-2.

6. The therapeutic agent for rheumatoid arthritis according to Claim 3, wherein the substances which inhibit the binding of an erbB ligand to c-erbB-2 or the substances which inhibit the binding of an erbB ligand to a receptor comprising c-erbB-2 are substances which bind to an erbB ligand and inhibit the binding of the erbB ligand to c-erbB-2 or a receptor forming a heteroreceptor with c-erbB-2.

7. The therapeutic agent for rheumatoid arthritis according to Claim 5, wherein the antagonists of c-erbB-2 or receptors forming heteroreceptors with c-erbB-2 are neutralizing antibodies recognizing c-erbB-2 or receptors forming heteroreceptors with c-erbB-2, fragments of said antibodies, or derivatives thereof.

8. The therapeutic agent for rheumatoid arthritis according to Claim 6, wherein the substances which bind to an erbB ligand and inhibit the binding of the erbB ligand to c-erbB-2 or a receptor forming a heteroreceptor with c-erbB-2 are neutralizing antibodies recognizing erbB ligands, fragments of said antibodies, or derivatives thereof.

9. The therapeutic agent for rheumatoid arthritis according to Claim 3, wherein the substances which inhibit the signal transduction after the binding of an erbB ligand to c-erbB-2 are substances which inhibit the tyrosine phosphorylation of c-erbB-2 or substances which inhibit the interaction between phosphorylated c-erbB-2 and an intracellular signaling molecule.

10. The therapeutic agent for rheumatoid arthritis according to Claim 9, wherein the substances which inhibit the tyrosine phosphorylation of c-erbB-2 are tyrosine kinase inhibitors.

11. The therapeutic agent for rheumatoid arthritis according to Claim 9, wherein the substances which inhibit the interaction between phosphorylated c-erbB-2 and an intracellular signaling molecule are antibodies to the intracellular domain of c-erbB-2, fragments of said antibodies, or derivatives thereof.

12. The therapeutic agent for rheumatoid arthritis according to Claim 2, wherein the substance which suppresses the expression of c-erbB-2 is at least one member selected from the group consisting of antisense DNAs, antisense oligonucleotides, triple helix-forming oligonucleotides and ribozymes for the c-erbB-2 gene.

13. A synoviocyte growth inhibitor comprising, as an active ingredient, a substance which inhibits the biological activities of c-erbB-2.

14. The synoviocyte growth inhibitor according to Claim 13, wherein the substance which inhibits the biological activities of c-erbB-2 is a substance which inhibits the signal transduction mediated by c-erbB-2 or a substance which suppresses the expression of c-erbB-2.

15. The synoviocyte growth inhibitor according to Claim 14, wherein the substance which inhibits the signal transduction mediated by c-erbB-2 is at least one member selected from the group consisting of substances which inhibit the binding of an erbB ligand to c-erbB-2, substances which inhibit the binding of a c-erbB-2 ligand to a receptor comprising c-erbB-2, substances which inhibit the dimerization of c-erbB-2, and substances which inhibit the signal transduction after the binding of an erbB ligand to c-erbB-2.

16. The synoviocyte growth inhibitor according to Claim 14, wherein the substance which inhibits the signal transduction mediated by c-erbB-2 is at least one member selected from the group consisting of neutralizing antibodies recognizing c-erbB-2, fragments of said antibodies, and derivatives thereof.

17. The synoviocyte growth inhibitor according to Claim 15, wherein the substances which inhibit the binding of an erbB ligand to c-erbB-2 or the substances which inhibit the binding of an erbB ligand to a receptor comprising c-erbB-2 are c-erbB-2 antagonists or antagonists of receptors forming heteroreceptors with c-erbB-2.

18. The synoviocyte growth inhibitor according to Claim 15, wherein the substances which inhibit the binding of an erbB ligand to c-erbB-2 or the substances which inhibit the binding of a c-erbB-2 ligand to a receptor comprising c-erbB-2 are substances which bind to an erbB ligand and inhibit the binding of the erbB ligand to c-erbB-2 or a receptor forming a heteroreceptor with c-erbB-2.

19. The synoviocyte growth inhibitor according to Claim 17, wherein the antagonists of c-erbB-2 or receptors forming heteroreceptors with c-erbB-2 are neutralizing antibodies recognizing c-erbB-2 or receptors forming heteroreceptors with c-erbB-2, fragments of said antibodies, or derivatives thereof.

20. The synoviocyte growth inhibitor according to Claim 18, wherein the substances which bind to an erbB ligand and inhibit the binding of the erbB ligand to c-erbB-2 or a receptor forming a heteroreceptor with c-erbB-2 are neutralizing antibodies recognizing erbB ligands, fragments of said antibodies, or derivatives thereof.

21. The synoviocyte growth inhibitor according to Claim 15, wherein the substances which inhibit the signal transduction after the binding of an erbB ligand to c-erbB-2 are substances which inhibit the tyrosine phosphorylation of c-erbB-2 or substances which inhibit the interaction between phosphorylated c-erbB-2 and an intracellular signaling molecule.

22. The synoviocyte growth inhibitor according to Claim 21, wherein the substances which inhibit the tyrosine phosphorylation of c-erbB-2 are tyrosine kinase inhibitors.

23. The synoviocyte growth inhibitor according to Claim 21, wherein the substances which inhibit the interaction between phosphorylated c-erbB-2 and an intracellular signaling molecule are antibodies to the intracellular domain of c-erbB-2, fragments of said antibodies, or derivatives thereof.

24. The synoviocyte growth inhibitor according to Claim 21, wherein the substance which inhibits the expression of c-erbB-2 is selected from the group consisting of antisense DNAs, antisense oligonucleotides, triple helix-forming oligonucleotides and ribozymes for the c-erbB-2 gene.

25. A diagnostic agent for rheumatoid arthritis comprising, as an active ingredient, a substance which specifically binds to c-erbB-2 or to an mRNA or cDNA for c-erbB-2.

26. The diagnostic agent for rheumatoid arthritis according to Claim 25, wherein the substance which binds to c-erbB-2 or to an mRNA or cDNA for c-erbB-2 is at least one member selected from the group consisting of antibodies recognizing c-erbB-2, DNAs of c-erbB-2 gene, fragments of said DNAs, and oligonucleotides consisting of 10 to 50 nucleotides identical to the nucleotide sequences of said DNAs or complementary strands thereof.

27. A diagnostic method for rheumatoid arthritis which comprises detecting or determining c-erbB-2 in synoviocytes by using the diagnostic agent according to Claim 25 or 26.
